# EUROPÄISCHE PATENTSCHRIFT

(11) **EP 2 468 716 B1**
(45) Veröffentlichungstag und Bekanntmachung des Hinweises auf die Patenterteilung: **19.07.2017**
(21) Anmeldenummer: 11009886.0
(22) Anmeldetag: 15.12.2011
(51) Int. Cl.: C07C 235/12, C07D 235/12, C07C 227/16, C07C 227/18

(54) **Prozess zur Herstellung von Bendamustinalkylester, Bendamustin sowie Derivaten davon**
Process for producing bendamustine alkyl esters, bendamustine and derivatives of same
Processus de fabrication d'ester d'alkyl de bendamustine, de bendamustine et de ses dérivés

(30) Priorität: 22.12.2010 DE 102010055499; 22.12.2010 US 201061426098 P
(43) Veröffentlichungstag der Anmeldung: 27.06.2012
(73) Patentinhaber: Heraeus Deutschland GmbH & Co. KG, 63450 Hanau (DE)
(72) Erfinder: Groh, Kai, Dr., 65428 Rüsselsheim (DE); Rauter, Holger, Dr., 36103 Flieden (DE); Born, Dirk, 63599 Biebergemünd (DE)
(74) Vertreter: Heraeus IP

(56) Entgegenhaltungen:
- DE-A1- 19 824 929
- WERNER W ET AL: "Synthesis of a potential metabolite of the anticancer drug bendamustine (CytostasanR)", DIE PHARMAZIE, GOVI VERLAG PHARMAZEUTISCHER VERLAG GMBH, ESCHBORN, DE, Bd. 46, Nr. 2, 1. Februar 1991 (1991-02-01), Seiten 113-114, XP008149662, ISSN: 0031-7144
- WERNER W ET AL: "Hydrolyseprodukte des Cancerostaticums Cytostasan (Bendamustin)", DIE PHARMAZIE, GOVI VERLAG PHARMAZEUTISCHER VERLAG GMBH, ESCHBORN, DE, Bd. 42, Nr. 4, 1. Januar 1987 (1987-01-01) , Seiten 272-273, XP008060249, ISSN: 0031-7144
- R. GUST ET.AL.: "Investigations on the stability of bendamustin, a cytostatic agent of the nitrogen mustard type, I. Synthesis, Isolation, and characterization of reference substances", MONATSHEFTE FÜR CHEMIE, Bd. 128, 1997, Seiten 291-299, XP002672681,

## Beschreibung

Die vorliegende Erfindung betrifft ein Verfahren zur Herstellung von Esterverbindungen, die durch die Formel (I) 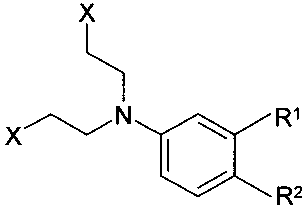 dargestellt sind, oder Salzen davon, wobei X für einen Halogenrest steht, R¹ und R² so gewählt sind, dass (1) R¹ H ist und R² -CH₂(CH₂)ₘCOOR³ ist, (2) R¹ -CH₂(CH₂)ₘCOOR³ ist und R² H ist oder (3) R¹ und R² zusammen für einen Rest stehen, der durch die Formel (II) 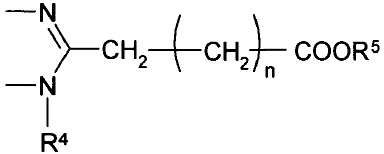 dargestellt ist, wobei R³, R⁴ und R⁵ unabhängig voneinander für einen Alkylrest stehen, und wobei m und n unabhängig voneinander eine Zahl im Bereich von 0-12 darstellen. Die vorliegende Erfindung betrifft ebenfalls ein Verfahren zur Herstellung des entsprechend hydrolysierten Esters, d. h. einer Verbindung, die die durch die Formel (VII) 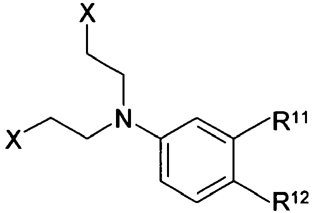 dargestellt ist, oder einem Salz davon, wobei X für einen Halogenrest steht, R¹¹ und R¹² so gewählt sind, dass (1) R¹¹ H ist und R¹² -CH₂(CH₂)ₘCOOH ist, (2) R¹¹ CH₂(CH₂)ₘCOOH ist und R¹² H ist oder (3) R¹¹ und R¹² zusammen für einen Rest stehen, der durch die Formel (VIII) 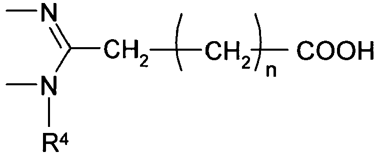 dargestellt ist, wobei R⁴ für einen Alkylrest steht und wobei m und n unabhängig voneinander eine Zahl im Bereich von 0 - 12 darstellen.

Verbindungen mit der oben genannten Struktur gemäß Formel (VII) werden zur Chemotherapie bei verschiedenen Krebserkrankungen eingesetzt. Ihre Wirkung beruht auf der Alkylierung der zelleigenen DNA oder RNA, wodurch es insbesondere zu einer Unterbindung der DNA-Replikation und in der Folge zur Apoptose der entsprechenden Zelle kommt. Die Einsatzgebiete der genannten Verbindungen umfassen vor allem die Therapie von verschiedenen Leukämien und Lymphomen, des Plasmozytoms und des Bronchialkarzinoms. Strukturell leiten sich diese Verbindungen vom Stickstoff-Lost ab.

Bendamustin 4-[5-[Bis(2-chlorethyl)amino]-1-methylbenzimidazol-2-yl]butansäure, das einen Vertreter dieser Gruppe darstellt, wurde erstmals im Jahr 1963 von Ozegowski und Krebs beschrieben (J. Prakt. Chem. 1963; 20: 178-86).

Ein vorteilhaftes Verfahren zur Herstellung von Bendamustin ist aus der DD 34727 bekannt. Die darin offenbarte Synthese erfolgt über den entsprechenden Bendamustinester, 4-[5-[Bis(2-chlorethyl)amino]-1-methylbenzimidazol-2-yl]butansäureester, der durch Chlorierung von 4-[5-[Bis(2-hydroxyethyl)amino]-1-methylbenzimidazol-2-yl]butansäureester hergestellt wird. Zur Substitution der beiden endständigen Hydroxylgruppen dieser Ausgangsverbindung durch Chlor wird Thionylchlorid verwendet. Mit dem in der DD 34727 beschriebenen Verfahren lässt sich tatsächlich in signifikanten Mengen Bendamustin erhalten. Nachteilig an diesem Verfahren ist allerdings, dass Bendamustin in sehr schwankenden Ausbeuten geliefert wird, die zudem noch bei Vergrößerung der Ansätze deutlich abfallen. Die Dokumente WERNER W ET AL, DIE PHARMAZIE, Bd. 46, Nr. 2, 1991, Seiten 113-114, sowie WERNER W ET AL: DIE PHARMAZIE, Bd. 42, Nr. 4, 1987, Seiten 272-273 lehren ebenfalls die Verwendung von Thionylchlorid als Chlorierungsmittel. Zur Lösung dieses Problems wird in der DD 159877 vorgeschlagen, das Reaktionsgemisch von Thionylchlorid und 4-[5-[Bis(2-hydroxyethyl)amino]-1-methylbenzimidazol-2-yl]butansäureester zum Abbruch der Reaktion direkt in wässrige Salzsäure einzurühren, wobei das überschüssige Thionylchlorid zersetzt wird. Durch diese Maßnahme konnte eine quantitative Steigerung der Ausbeuten an Bendamustinester und letztlich auch Bendamustin erreicht werden.

In weiteren Versuchen stellte sich jedoch heraus, dass selbst mit dem Verfahren der DD 159877 eine Steigerung der Ausbeute an Bendamustinester bzw. Bendamustin auf mehr als 80 % nicht zu erreichen ist. Darüber hinaus wurde festgestellt, dass bei diesem Verfahren Nebenprodukte entstehen, die sich von Bendamustinester bzw. Bendamustin nur schwer abtrennen lassen und so die Produktqualität negativ beeinflussen. Die erhaltenen Ausbeuten an Bendamustinester bzw. Bendamustin ließen sich zudem nicht reproduzierbar erhalten, sondern schwankten trotz Einhaltung gleicher Versuchsbedingungen erheblich. Dies zeigte sich insbesondere beim Versuch, das Verfahren der DD 159877 im industriellen Maßstab durchzuführen. Hier erwies sich die Reaktionssteuerung aufgrund des notwendigen hohen Überschusses an Thionylchlorid als schwierig. Ein weiteres Problem ergibt sich aus prozessökonomischen Gesichtspunkten aufgrund der Tatsache, dass bei der Verwendung von Thionylchlorid große Mengen saurer Prozessabgase entstehen, was hohe Anforderungen an die Abluftreinigung stellt. Ähnliche Probleme ergeben sich, wenn das Verfahren der DD 159877 auf die Herstellung anderer Verbindungen, die durch die Formeln (I) bzw. (VII) darstellbar sind, übertragen wird.

In Anbetracht des Stands der Technik bestand die der vorliegenden Erfindung zugrunde liegenden Aufgabe darin, ein Verfahren bereitzustellen, dass die Herstellung von Verbindungen mit den vorstehend gezeigten Strukturen in reproduzierbar hohen Ausbeuten ermöglicht. Das Verfahren sollte ferner einfach auch im industriellen Maßstab durchführbar sein. Zudem sollten bei diesem Verfahren keine großen Mengen an sauren Prozessabgasen entstehen.

Diese Aufgabe wurde gelöst durch die Verfahren der unabhängigen Ansprüche.

Die Erfindung stellt somit ein Verfahren zur Herstellung einer Verbindung, die durch die Formel (I) 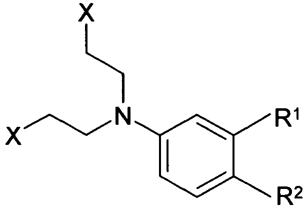 dargestellt ist, oder einem Salz davon, bereit, wobei X für einen Halogenrest steht, R¹ und R² so gewählt sind, dass (1) R¹ H ist und R²-CH₂(CH₂)ₘCOOR₃ ist, (2) R¹ -CH₂(CH₂)ₘCOOR³ ist und R² H ist oder (3) R¹ und R² zusammen für einen Rest stehen, der durch die Formel (II) 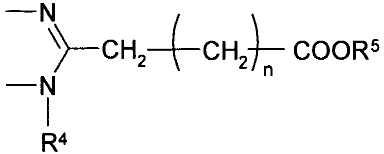 dargestellt ist, wobei R³, R⁴ und R⁵ unabhängig voneinander für einen Alkylrest stehen, und wobei m und n unabhängig voneinander eine Zahl im Bereich von 0-12 darstellen, wobei man eine Verbindung, die durch die Formel (III) 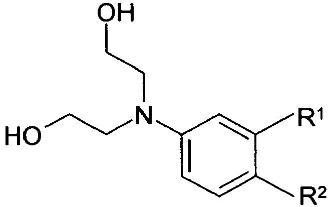 dargestellt ist, mit einer Mischung zur Reaktion bringt, die eine Verbindung (i), die aus der Gruppe ausgewählt ist, die aus einer Verbindung, die durch die Formel (IV) 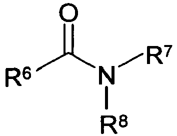 dargestellt ist, wobei R⁶ für H oder einen Alkylrest steht, und R⁷ und R⁸ unabhängig voneinander für einen Alkylrest stehen, und einer Verbindung, die durch die Formel (V) 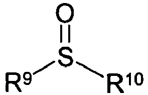 dargestellt ist, wobei R⁹ und R¹⁰ unabhängig voneinander für einen Alkylrest stehen, besteht, und eine Verbindung (ii), die durch die Formel (VI) 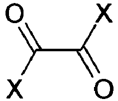 dargestellt ist, enthält.

Die Erfindung stellt ferner ein Verfahren zur Herstellung einer Verbindung, die durch die Formel (VII) 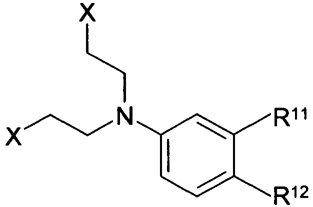 dargestellt ist, oder einem Salz davon, bereit, wobei X für einen Halogenrest steht, R¹¹ und R¹² so gewählt sind, dass (1) R¹¹ H ist und R¹² -CH₂(CH₂)ₘCOOH ist, (2) R¹¹ -CH₂(CH₂)ₘCOOH ist und R¹² H ist oder (3) R¹¹ und R¹² zusammen für einen Rest stehen, der durch die Formel (VIII) 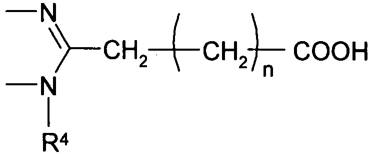 dargestellt ist, wobei R⁴ für einen Alkylrest steht und wobei m und n unabhängig voneinander eine Zahl im Bereich von 0-12 darstellen, wobei man das vorstehend beschriebene Verfahren durchführt und die erhaltene Esterverbindung, die durch die Formel (I) dargestellt ist, einer Esterhydrolyse unterzieht.

Überraschenderweise wurde festgestellt, dass durch die Verwendung einer Mischung umfassend wenigstens eine der Verbindungen gemäß den Formeln (IV) und (V) sowie der Verbindung gemäß Formel (VI) die endständigen Hydroxylgruppen in der Verbindung gemäß Formel (I) unter milden Bedingungen und bei moderaten Reaktionszeiten selektiv durch Halogenidgruppen substituiert werden können, wobei das gewünschte Produkt reproduzierbar in hohen Ausbeuten erhalten wird. Ohne an eine Theorie gebunden sein zu wollen, läuft diese Reaktion wohl über ein zwischenzeitlich gebildetes aktiviertes Imminiumion ab, das aus der Verbindung gemäß Formel (VI) oder (V) sowie der Verbindung gemäß Formel (VI) gebildet wird.

Das erfindungsgemäße Verfahren umfasst einerseits die Herstellung einer Verbindung, die durch die Formel (I) 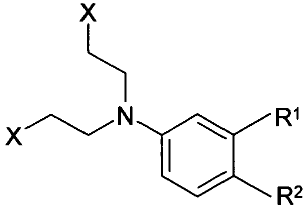 dargestellt ist, oder einem Salz davon.

In Formel (I) steht der Rest X für einen Halogenrest. Bei dem Halogenrest kann es sich beispielsweise um einen Chlorrest oder einen Bromrest handeln. Erfindungsgemäß stehen beide Reste X in Formel (I) für denselben Substituenten. Gemäß einer besonders bevorzugten Ausführungsform sind diese Halogenreste Chlorreste.

Die Reste R¹ und R² können unterschiedliche Bedeutungen annehmen.

Gemäß einer ersten Alternative (1) handelt es sich bei dem Rest R¹ um Wasserstoff. In diesem Fall steht der Rest R² für den Rest -CH₂(CH₂)ₘCOOR³. In diesem Rest steht R³ für einen Alkylrest. Bei dem Alkylrest kann es sich um einen verzweigten oder unverzweigten Alkylrest handeln. Gemäß einer besonders bevorzugten Ausführungsform ist dieser Alkylrest ein unverzweigter Alkylrest. Die Kettenlänge dieses Alkylrestes ist nicht weiter eingeschränkt. Beispielsweise kann dieser Alkylrest 1-20 Kohlenstoffatome, so zum Beispiel 1-12 Kohlenstoffatome, 1-8 Kohlenstoffatome, 1-4 Kohlenstoffatome oder 1 oder 2 Kohlenstoffatome, aufweisen. Der Alkylrest kann insbesondere ein Methylrest, ein Ethylrest, ein Propylrest oder ein Butylrest sein. Der Index m kann im Rest R² eine ganze Zahl im Bereich von 0-12, vorzugsweise im Bereich von 0-10, noch mehr bevorzugt im Bereich von 0-8, ganz besonders bevorzugt im Bereich von 0-6 und insbesondere im Bereich von 0-3 einnehmen. Beispielsweise kann der Index m die Zahl 2 einnehmen.

Gemäß einer zweiten Alternative (2) handelt es sich bei dem Rest R² um Wasserstoff. In diesem Fall steht der Rest R¹ für den Rest -CH₂(CH₂)ₘCOOR³. In diesem Rest steht R³ für einen Alkylrest. Bei dem Alkylrest kann es sich um einen verzweigten oder unverzweigten Alkylrest handeln. Gemäß einer besonders bevorzugten Ausführungsform ist dieser Alkylrest ein unverzweigter Alkylrest. Die Kettenlänge dieses Alkylrestes ist nicht weiter eingeschränkt. Beispielsweise kann dieser Alkylrest 1 - 20 Kohlenstoffatome, so zum Beispiel 1-12 Kohlenstoffatome, 1-8 Kohlenstoffatome, 1-4 Kohlenstoffatome oder 1 oder 2 Kohlenstoffatome, aufweisen. Der Alkylrest kann insbesondere ein Methylrest, ein Ethylrest, ein Propylrest oder ein Butylrest sein. Der Index m kann im Rest R₂ eine ganze Zahl im Bereich von 0-12, vorzugsweise im Bereich von 0-10, noch mehr bevorzugt im Bereich von 0-8, ganz besonders bevorzugt im Bereich von 0-6 und insbesondere im Bereich von 0-3 einnehmen. Beispielsweise kann der Index m die Zahl 2 einnehmen.

Gemäß einer dritten Alternative (3) bilden der Rest R¹ und der Rest R² einen Rest, der durch die Formel (II) 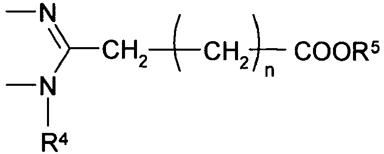 dargestellt ist. Demnach steht diese Alternative für ein heterozyklisches Ringsystem mit Benzimidazolstruktur. Diese kann durch die nachstehende Formel (IX) 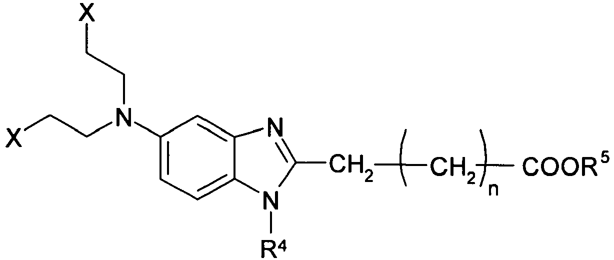 wiedergegeben werden. Der Rest R⁴ steht dabei für einen Alkylrest. Bei dem Alkylrest R⁴ kann es sich um einen verzweigten, vorzugsweise aber um einen unverzweigten Alkylrest handeln. Die Kettenlänge dieses Alkylrestes ist nicht weiter eingeschränkt. Beispielsweise kann dieser Alkylrest 1 - 20 Kohlenstoffatome, so zum Beispiel 1-12 Kohlenstoffatome, 1 - 8 Kohlenstoffatome, 1 - 4 Kohlenstoffatome oder 1 oder 2 Kohlenstoffatome, aufweisen. Der Alkylrest kann insbesondere ein Methylrest, ein Ethylrest, ein Propylrest oder ein Butylrest sein. Der Rest R⁵ steht ebenfalls für einen Alkylrest. Bei dem Alkylrest R⁵ kann es sich ebenso um einen verzweigten, vorzugsweise aber um einen unverzweigten Alkylrest handeln. Die Kettenlänge dieses Alkylrestes ist nicht weiter eingeschränkt. Beispielsweise kann dieser Alkylrest 1-20 Kohlenstoffatome, so zum Beispiel 1-12 Kohlenstoffatome, 1-8 Kohlenstoffatome, 1-4 Kohlenstoffatome oder 1 oder 2 Kohlenstoffatome, aufweisen. Der Alkyrest kann insbesondere ein Methylrest, ein Ethylrest, ein Propylrest oder ein Butylrest sein. Der Index n kann eine ganze Zahl im Bereich von 0-10 darstellen. Vorzugsweise steht n für eine ganze Zahl im Bereich von 0-8, mehr bevorzugt für eine ganze Zahl im Bereich von 0-6, noch mehr bevorzugt für eine ganze Zahl im Bereich von 0-4 und besonders bevorzugt für eine ganze Zahl im Bereich von 0 - 3. Beispielsweise kann der Index n die Zahl 2 einnehmen.

Gemäß einer bevorzugten Ausführungsform handelt es sich bei der Verbindung gemäß Formel (I) um 4-[5-[Bis(2-chlorethyl)aminol-1-methylbenzimidazol-2-yl]butansäurealkylester oder 4-[Bis(2-chlorethyl)amino]benzenbutansäurealkylester. Gemäß einer besonders bevorzugten Ausführungsform handelt es sich bei der Verbindung gemäß Formel (I) um 4-[5-[Bis(2-chlorethyl)amino]-1-methylbenzimidazol-2-yl]butansäureethylester oder 4-[Bis(2-chlorethyl)amino]benzenbutansäureethylester.

Erfindungsgemäß kann nicht nur der vorstehend beschriebene Ester, sondern auch ein Salz davon hergestellt werden. In diesem Salz ist vorzugsweise wenigstens eines der Stickstoffatome der Verbindung gemäß Formel (I), insbesondere das Stickstoffatom, das nicht Teil der Ringstruktur ist, protoniert. Der protonierte Ester liegt dann in Verbindung mit einem entsprechenden Anion vor. Bei diesem Anion handelt es sich vorzugsweise um ein Halogenidion. Gemäß einer besonders bevorzugten Ausführungsform liegt der Ester als Hydrochlorid vor. Gemäß einer ganz besonders bevorzugten Ausführungsform handelt es sich dabei um das Hydrochlorid von 4-[5-[Bis(2-chlorethyl)amino]-1-methylbenzimidazol-2-yl]butansäurealkylester oder 4-[Bis(2-chlorethyl)amino]benzenbutansäurealkylester, wie zum Beispiel 4-[5-[Bis(2-chlorethyl)amino]-1-methylbenzimidazol-2-yl]butansäureethylester oder 4-[Bis(2-chlorethyl)amino]benzenbutansäureethylester.

Als Edukt zur Herstellung der Verbindung gemäß Formel (I) dient eine Verbindung, die durch die Formel (III) 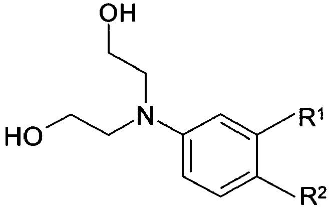 dargestellt ist. Dabei nehmen die Reste R¹, R², R³, R⁴ und R⁵, sowie die Indizes m und n die ihnen vorstehend zugeschriebene Bedeutung ein. Gemäß einer bevorzugten Ausführungsform handelt es sich bei diesem Edukt um 4-[5-[Bis(2-hydroxyethyl)amino]-1-methylbenzimidazol-2-yl]butansäurealkylester oder 4-[Bis(2-hydroxyethyl)amino]benzenbutansäurealkylester, insbesondere um 4-[5-[Bis(2-hydroxyethyl)amino]-1-methylbenzimidazol-2-yl]butansäureethylester oder 4-[Bis(2-hydroxyethyl)amino]benzenbutansäureethylester.

Dieses Edukt wird mit einer Mischung zur Reaktion gebracht, die wenigstens eine Verbindung (i) und eine Verbindung (ii) enthält.

Verbindung (i) ist aus der Gruppe ausgewählt, die aus einer Verbindung, die durch die Formel (IV) 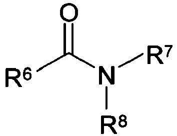 dargestellt ist, und einer Verbindung, die durch die Formel (V) 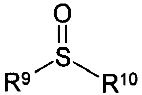 dargestellt ist, besteht.

In der Verbindung gemäß Formel (IV) steht der Rest R⁶ für Wasserstoff oder einen Alkylrest. Als Alkylrest können verzweigte, bevorzugt aber unverzweigte Alkylreste in Betracht kommen. Diese Alkylreste können vorzugsweise 1-12 Kohlenstoffatome, mehr bevorzugt 1-10 Kohlenstoffatome, noch mehr bevorzugt 1 - 6 Kohlenstoffatome, besonders bevorzugt 1-4 Kohlenstoffatome, ganz besonders bevorzugt 1-2 Kohlenstoffatome und insbesondere ein Kohlenstoffatom umfassen.

Die Reste R⁷ und R⁸ stehen unabhängig voneinander für Alkylreste. Auch hier können als Alkylrest verzweigte, bevorzugt aber unverzweigte Alkylreste in Betracht kommen. Diese Alkylreste können vorzugsweise 1-12 Kohlenstoffatome, mehr bevorzugt 1-10 Kohlenstoffatome, noch mehr bevorzugt 1-6 Kohlenstoffatome, besonders bevorzugt 1-4 Kohlenstoffatome und ganz besonders bevorzugt 1 - 2 Kohlenstoffatome umfassen.

Besonders bevorzugte Verbindungen gemäß Formel (V) sind Dimethylformamid (DMF) und Dimethylacetamid (DMAc).

Die Reste R⁹ und R¹⁰ in Formel (V) stehen unabhängig voneinander für Alkylreste. Als Alkylrest können wiederum verzweigte, bevorzugt aber unverzweigte Alkylreste in Betracht kommen. Diese Alkylreste können vorzugsweise 1-12 Kohlenstoffatome, mehr bevorzugt 1-10 Kohlenstoffatome, noch mehr bevorzugt 1 - 6 Kohlenstoffatome, besonders bevorzugt 1 - 4 Kohlenstoffatome, ganz besonders bevorzugt 1-2 Kohlenstoffatome und insbesondere ein Kohlenstoffatom umfassen. Eine bevorzugte Verbindung gemäß Formel (V) ist Dimethylsulfoxid (DMSO).

Bei Verbindung (ii) handelt es sich um eine Verbindung, die durch die Formel (VI) 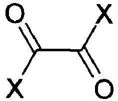 dargestellt ist, wobei X für einen Halogenrest, vorzugsweise für Chlor, steht. Dementsprechend ist als Verbindung gemäß Formel (VI) Oxalylchlorid bevorzugt.

Gemäß einer bevorzugten Ausführungsform der Erfindung wird ein 4-[5-[Bis(2-chlorethyl)amino]-1-methylbenzimidazol-2-yl]butansäurealkylester oder ein Salz hiervon durch Umsetzung von 4-[5-[Bis(2-hydroxyethyl)amino]-1-methylbenzimidazol-2-yl]butansäurealkylester mit Oxalylchlorid und Dimethylformamid hergestellt. Gemäß einer besonders bevorzugten Ausführungsform wird ein 4-[5-[Bis(2-chlorethyl)amino]-1-methylbenzimidazol-2-yl]butansäureethylester oder ein Hydrochlorid hiervon hergestellt, indem 4-[5-[Bis(2-hydroxyethyl)amino]-1-methylbenzimidazol-2-yl]butansäureethylester mit Oxalylchlorid und Dimethylformamid zur Reaktion gebracht wird.

Die Verbindung (ii), die durch die Formel (VI) dargestellt ist, wird wenigstens äquimolar, vorzugsweise jedoch im molaren Überschuss zur Verbindung gemäß Formel (III) eingesetzt. Gemäß einer besonders bevorzugten Ausführungsform beträgt das molare Verhältnis von der Verbindung gemäß Formel (VI) zur Verbindung gemäß Formel (III) wenigstens 1,3 und noch mehr bevorzugt wenigstens 1,5.

In einer bevorzugten Ausgestaltungsform der Erfindung werden zunächst die Verbindungen (i) (d.h. die Verbindungen gemäß den Formeln (IV) und/oder (V)) und (ii) (d.h. die Verbindung gemäß Formel (VI)) miteinander vereinigt und anschließend mit der Verbindung gemäß Formel (III) in Kontakt gebracht. Hierzu liegen die Verbindungen (i) und (ii) sowie die Verbindung gemäß Formel (III) sowohl einzeln als auch vereinigt vorzugsweise in einem geeigneten Lösungsmittel gelöst vor. Als Lösungsmittel können beispielsweise halogenierte Kohlenwasserstoffe, wie Dichlormethan oder Chloroform, oder weitere aprotische Lösungsmittel, wie Tetrahydrofuran oder Dioxan, zum Einsatz kommen.

Vorzugsweise wird beim erfindungsgemäßen Verfahren die in einem geeigneten Lösungsmittel gelöste Verbindung gemäß Formel (VI) in einem Reaktionsgefäß vorgelegt und gegebenenfalls gekühlt, wobei sich Temperaturen im Bereich von 0 - 10°C, so zum Beispiel Temperaturen im Bereich von 1 - 5°C oder im Bereich von 1 - 3°C, als besonders vorteilhaft erwiesen haben.

Auch die Verbindung gemäß Formel (IV) oder Formel (V) wird vorzugsweise in einem geeigneten Lösungsmittel gelöst und anschließend gegebenenfalls abgekühlt. Dabei erwiesen sich auch hier Temperaturen im Bereich von 0 - 10°C, so zum Beispiel Temperaturen im Bereich von 1 - 5°C oder im Bereich von 1 - 3°C, als besonders vorteilhaft.

Gemäß einer möglichen Ausführungsform der Erfindung werden in einem nächsten Schritt die Verbindung gemäß Formel (VI) und die Verbindung gemäß Formel (IV) oder (V) vereinigt. Dies kann beispielsweise dadurch erfolgen, dass die Lösung, die die Verbindung gemäß Formel (VI) enthält, zur Lösung, die die Verbindung gemäß Formel (IV) oder (V) enthält, zudosiert wird, so zum Beispiel zugetropft, wird. Selbstverständlich kann jedoch auch die Lösung, die die Verbindung gemäß Formel (IV) oder (V) enthält, zur Lösung, die die Verbindung gemäß Formel (VI) enthält, zudosiert, so zum Beispiel zugetropft, werden. In diesem Schritt wird demnach vorzugsweise eine Lösung erhalten, die die Verbindungen (i) und (ii) umfasst.

Diese Lösung wird nun vorzugsweise mit einer Lösung versetzt, die die Verbindung gemäß Formel (III) enthält. Hierzu kann beispielsweise die Lösung, die die Verbindung gemäß Formel (III) enthält, zur Lösung, die die Verbindungen (i) und (ii) enthält, zugetropft werden.

Die Umsetzung der in der so erhaltenen Reaktionsmischung enthaltenen Verbindungen kann anschließend durch eine dem Fachmann geläufige Weise gesteuert werden.

Beispielsweise kann das Reaktionsgemisch unter Rückfluss gerührt werden.

Das Rühren der Reaktionsmischung unter Rückfluss kann zum Beispiel für einen Zeitraum von wenigstens einer Stunde, vorzugsweise für einen Zeitraum von wenigstens zwei Stunden, noch mehr bevorzugt für einen Zeitraum von wenigstens vier Stunden und besonders bevorzugt für einen Zeitraum von wenigstens sechs Stunden erfolgen. Gemäß einer bevorzugten Ausführungsform erfolgt das Rühren der Reaktionsmischung unter Rückfluss für einen Zeitraum von 1 - 24 Stunden, mehr bevorzugt für einen Zeitraum von 2-20 Stunden, noch mehr bevorzugt für einen Zeitraum von 4-16 Stunden und besonders bevorzugt für einen Zeitraum von 6 - 12 Stunden.

Die Reaktion kann gegebenenfalls in einer Inertgasatmosphäre, so zum Beispiel in einer Stickstoffatmosphäre oder in einer Edelgasatmosphäre, durchgeführt werden.

Das Reaktionsprodukt liegt nach der Umsetzung, je nach pH-Wert der Reaktionsmischung, als freie Base oder als Salz vor. Handelt es sich beispielsweise bei dem Reaktionsprodukt gemäß Formel (I) um Bendamustinethylester, so liegt dieses bei pH-Werten von ≥ 7,5 als freie Base, bei niedrigeren pH-Werten als Salz, beispielsweise als Hydrochlorid, vor. Insofern ist es möglich, durch Einstellung des pH-Wertes auf einen geeigneten Wert das Reaktionsprodukt gemäß Formel (I) als freie Base oder aber als Salz zu erhalten.

Nach der Umsetzung kann das Reaktionsprodukt, das durch die Formel (I) dargestellt ist, gemäß einer ersten Ausführungsform aus dem Reaktionsgemisch isoliert werden. Alternativ dazu kann gemäß einer zweiten Ausführungsform das Reaktionsgemisch aber auch unmittelbar zur Durchführung einer Esterhydrolyse verwendet werden, um das Produkt gemäß Formel (VII) herzustellen.

Wenn eine Isolierung des Reaktionsprodukts gemäß Formel (I) aus der Reaktionsmischung gewünscht ist, so kann dies auf herkömmliche Weise erfolgen. Beispielsweise kann die Reaktionsmischung mit Wasser versetzt werden, um eine organische und eine wässrige Phase zu erzeugen. Während die organische Phase das Reaktionsprodukt gemäß Formel (I) enthält, dient die wässrige Phase dazu, wasserlösliche Bestandteile, wie zum Beispiel Salze, aus der organischen Phase zu entfernen.

In einem nächten Schritt kann, falls gewünscht oder erforderlich, der pH-Wert eingestellt werden, um das Reaktionsprodukt gemäß Formel (I) in die freie Base oder aber ein entsprechendes Salz umzuwandeln. Liegt das Reaktionsprodukt in der Reaktionsmischung beispielsweise als Salz vor, so kann durch Zugabe einer Base der pH-Wert der Reaktionsmischung erhöht werden, um das Salz in die freie Base umzuwandeln. Dies kann zum Beispiel durch Zugabe basischer Lösungen, wie Kaliumcarbonat, zu der mit Wasser versetzten Reaktionsmischung erfolgen.

Anschließend wird das Gemisch aus der Reaktionsmischung und Wasser vorzugsweise intensiv gemischt, um ein Übertreten von wasserlöslichen Bestandteilen aus der organischen Phase in die wässrige Phase zu ermöglichen. Die organische Phase wird danach vorzugsweise aus dem Gemisch entfernt und aufgehoben, während die wässrige Phase mit einem organischen Lösungsmittel, so zum Beispiel mit dem oder einem der Lösungsmittel, das zur Lösung der Verbindungen gemäß Formel (III) oder den Verbindungen (i) bzw. (ii) vor der Umsetzung gedient hat, versetzt und extrahiert wird. Die organische Phase bzw. die vereinigten organischen Phase können danach beispielsweise im Vakuum bis zur Gewichtskonstanz getrocknet werden, um das Reaktionsprodukt gemäß Formel (I) zu erhalten.

Das Reaktionsprodukt gemäß Formel (I) dient üblicherweise als Intermediat zur Herstellung einer Verbindung, die durch die Formel (VII) 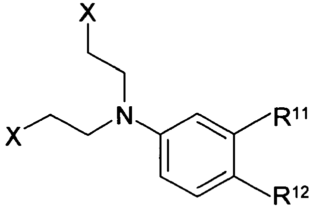 dargestellt ist, oder einem Salz davon.

In Formel (VII) steht X wiederum für ein Halogenatom, vorzugsweise für ein Chloratom oder ein Bromatom und besonders bevorzugt für ein Chloratom.

Die Reste R¹¹ und R¹² können verschiedene Bedeutungen einnehmen.

Gemäß einer ersten Alternative (1) handelt es sich bei dem Rest R¹¹ um Wasserstoff. In diesem Fall steht der Rest R¹² für den Rest -CH₂(CH₂)ₘCOOH. Der Index m kann im Rest R¹² eine ganze Zahl im Bereich von 0-12, vorzugsweise im Bereich von 0-10, noch mehr bevorzugt im Bereich von 0-8, ganz besonders bevorzugt im Bereich von 0-6 und insbesondere im Bereich von 0-3 einnehmen. Beispielsweise kann der Index m die Zahl 2 einnehmen.

Gemäß einer zweiten Alternative (2) handelt es sich bei dem Rest R¹² um Wasserstoff. In diesem Fall steht der Rest R¹¹ für den Rest -CH₂(CH₂)ₘCOOH. Der Index m kann im Rest R¹² eine ganze Zahl im Bereich von 0-12, vorzugsweise im Bereich von 0-10, noch mehr bevorzugt im Bereich von 0-8, ganz besonders bevorzugt im Bereich von 0-6 und insbesondere im Bereich von 0-3 einnehmen. Beispielsweise kann der Index m die Zahl 2 einnehmen.

Gemäß einer dritten Alternative (3) bilden der Rest R¹¹ und der Rest R¹² einen Rest, der durch die Formel (VIII) 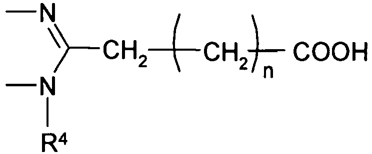 dargestellt ist. Demnach steht diese Alternative für ein heterozyklisches Ringsystem mit Benzimidazolstruktur. Diese kann durch die nachstehende Formel (X) 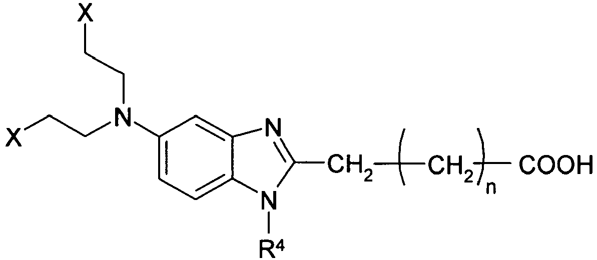 wiedergegeben werden. Der Rest R⁴ steht dabei für einen Alkylrest. Bei dem Alkylrest R⁴ kann es sich um einen verzweigten, vorzugsweise aber um einen unverzweigten Alkylrest handeln. Die Kettenlänge dieses Alkylrestes ist nicht weiter eingeschränkt. Beispielsweise kann dieser Alkylrest 1-20 Kohlenstoffatome, so zum Beispiel 1-12 Kohlenstoffatome, 1-8 Kohlenstoffatome, 1-4 Kohlenstoffatome oder 1 oder 2 Kohlenstoffatome, aufweisen. Der Alkylrest kann insbesondere ein Methylrest, ein Ethylrest, ein Propylrest oder ein Butylrest sein. Der Index n kann eine ganze Zahl im Bereich von 0 - 10 darstellen. Vorzugsweise steht n für eine ganze Zahl im Bereich von 0-8, mehr bevorzugt für eine ganze Zahl im Bereich von 0-6, noch mehr bevorzugt für eine ganze Zahl im Bereich von 0-4 und besonders bevorzugt für eine ganze Zahl im Bereich von 0-3. Beispielsweise kann der Index n die Zahl 2 einnehmen.

Gemäß einer bevorzugten Ausführungsform handelt es sich bei der Verbindung gemäß Formel (I) um 4-[5-[Bis(2-chlorethyl)amino]-1-methylbenzimidazol-2-yl]butansäure oder 4-[Bis(2-chlorethyl)amino]benzenbutansäure.

Erfindungsgemäß kann nicht nur die vorstehend beschriebene Säure, sondern auch ein Salz davon hergestellt werden. In diesem Salz ist vorzugsweise wenigstens eines der Stickstoffatome der Verbindung gemäß Formel (I), insbesondere das Stickstoffatom, das nicht Teil der Ringstruktur ist, protoniert. Der protonierte Ester liegt dann in Verbindung mit einem entsprechenden Anion vor. Bei diesem Anion handelt es sich vorzugsweise um ein Halogenidion. Gemäß einer besonders bevorzugten Ausführungsform liegt der Ester als Hydrochlorid vor. Gemäß einer ganz besonders bevorzugten Ausführungsform handelt es sich dabei um ein Hydrochlorid von 4-[5-[Bis(2-chlorethyl)amino]-1-methylbenzimidazol-2-yl]butansäure oder 4-[Bis(2-chlorethyl)amino]benzenbutansäure.

Zur Herstellung der Verbindung gemäß Formel (VII) wird zunächst das vorstehend beschriebene Verfahren zur Herstellung der Verbindung gemäß Formel (I) durchgeführt und das erhaltene Reaktionsprodukt einer Esterhydrolyse unterzogen. Dazu kann das erhaltene Reaktionsprodukt entweder, wie vorstehend ausgeführt, zunächst isoliert und danach hydrolysiert oder aber unmittelbar (d.h. ohne vorherige Isolierung) zur Säure umgesetzt werden.

Gemäß einer besonders bevorzugten Ausführungsform wird im Rahmen der Erfindung 4-[5-[Bis(2-chlorethyl)amino]-1-methylbenzimidazol-2-yl]butansäure oder ein Salz hiervon dadurch hergestellt, dass in einem ersten Schritt ein 4-[5-[Bis(2-hydroxyethyl)amino]-1-methylbenzimidazol-2-yl]butansäurealkylester oder ein Salz hiervon mit Oxalylchlorid und Dimethylformamid unter Bildung eines 4-[5-[Bis(2-chlorethyl)amino]-1-methylbenzimidazol-2-yl]butansäurealkylesters oder eines Salzes hiervon umgesetzt wird, und in einem zweiten Schritt der erhaltene 4-[5-[Bis(2-chlorethyl)amino]-1-methylbenzimidazol-2-yl]butansäurealkylester oder ein Salz hiervon hydrolysiert wird.

Zur Esterhydrolyse wird das Reaktionsprodukt gemäß Formel (I) vorzugsweise mit einer Säure versetzt. Als Säure dient dabei vorzugsweise eine anorganische Säure, insbesondere Salzsäure. Die Säure wird üblicherweise als konzentrierte Säure, zum Beispiel als konzentrierte Salzsäure (32 %), eingesetzt. Durch die auf die Säurezugabe zurückzuführende pH-Wert-Erniedrigung wird das bei stärker basischen pH-Werten instabile Reaktionsprodukt gemäß Formel (I) stabilisiert.

Das Reaktionsprodukt gemäß Formel (I) kann mit der Säure versetzt werden, indem die Säure mit dem isolierten Reaktionsprodukt gemäß Formel (I), mit einer Lösung des isolierten Reaktionsprodukts gemäß Formel (I) in einem geeigneten Lösungsmittel oder mit der vorstehend beschriebenen Reaktionsmischung, die das isolierte Reaktionsprodukt gemäß Formel (I) enthält, vereinigt wird.

Im Anschluss wird die erhaltene Mischung vorzugsweise bei einer Temperatur im Bereich von 10 - 80°C, mehr bevorzugt bei einer Temperatur im Bereich von 15 - 70°C und noch mehr bevorzugt bei einer Temperatur im Bereich von 20 - 60°C gerührt.

Die Reaktionsdauer beträgt vorzugsweise 30 Minuten bis sechs Stunden, mehr bevorzugt eine Stunde bis vier Stunden und noch mehr bevorzugt eine Stunde bis drei Stunden.

Nach der Umsetzung können gegebenenfalls in der Mischung enthaltene organische Bestandteile, wie zum Beispiel Lösungsmittel oder Lösungsmittelreste, entfernt werden. Dies kann vorzugsweise durch Destillation dieser organischen Bestandteile geschehen.

In einem weiteren optionalen Schritt wird die Mischung, die neben der Säure den nun hydrolysierten Ester gemäß Formel (VII) enthält, mit Aktivkohle versetzt und gerührt. Die Aktivkohle kann dazu dienen, eventuelle in der Mischung enthaltene Verunreinigungen absorptiv zu binden. Die Aktivkohle kann anschließend von der restlichen Mischung bspw. durch Filtration abgetrennt werden.

Die in der Mischung enthaltene Säure wird dann auf herkömmliche Weise von dem hydrolysierten Ester gemäß Formel (VII) entfernt. Vorzugsweise wird hierzu die Säure von dem hydrolysierten Ester gemäß Formel (VII) destillativ abgetrennt. Als Rückstand verbleibt die Verbindung gemäß Formel (VII), die beispielsweise als Feststoff oder als ölige Flüssigkeit vorliegen kann.

Der Rückstand kann, falls erforderlich, weiter aufgereinigt werden. Liegt der Rückstand beispielsweise als ölige Flüssigkeit vor, so kann zunächst mit einer geeigneten Lösung eine Fällung der Verbindung gemäß Formel (VII) herbeigeführt werden. Als Fällungslösung kommen insbesondere Gemische in Betracht, die Wasser und ferner ein Keton oder einen Alkohol enthalten. Aceton hat sich hierbei als geeignetes Keton, Ethanol als geeigneter Alkohol erwiesen. Nach der Fällung kann der erhaltene Rückstand von der Fällungslösung getrennt werden.

Im Anschluss kann der verbleibende Rückstand, falls erforderlich, mit einer geeigneten Waschlösung gewaschen und danach getrocknet werden. Als Waschlösung können vorzugsweise Wasser und Ethylacetat eingesetzt werden.

Die Erfindung wird durch die nachstehenden Beispiele beschrieben, die jedoch nicht als beschränkend zu verstehen sind.

### Ausführungsbeispiel 1a: Herstellung von Bendamustinethylester

26,7 g Oxalylchlorid (1,6 Äquivalente) wurden in 279 ml Dichlormethan gelöst und auf 1 °C abgekühlt. Bei 1 °C wurde ein Gemisch aus 17 ml Dimethylformamid (DMF) und 50 ml trockenem Dichlormethan langsam zudosiert. Dem so erhaltenen Reaktionsgemisch wurde eine Lösung aus 23 g Ethyl-4-(5-(bis-(2-hydroxyethyl)-amino)-1-methyl-1 H-benzo[d]imidazol-2-yl)butanoat in 115 ml Dichlormethan zugesetzt. Das Reaktionsgemisch wurde 8 h unter Rückfluss (45°C Manteltemperatur) gerührt, abgekühlt und anschließend mit 50 ml Reinstwasser versetzt. Durch Zugabe von 25%iger K₂CO₃-Lösung wurde der pH-Wert auf pH 8 - 9,5 einjustiert. Nach intensivem Durchmischen wurde die organische Phase abgetrennt und die wässrige Phase mit weiteren 83 ml Dichlormethan extrahiert. Die vereinigten organischen Phasen wurden im Vakuum bis zur Gewichtskonstanz getrocknet.

Dieser Versuch wurde sechsmal durchgeführt. Es wurden folgende Ausbeuten an Bendamustinethylester erhalten:

| V1a-1 | V1a-2 | V1a-3 | V1a-4 | V1a-5 | V1a-6 | Mittlere Ausbeute | Maximale Abweichung von mittlerer Ausbeute |
|---|---|---|---|---|---|---|---|
| 93,2% | 92,7% | 92,5% | 90,4% | 93,4% | 93,0% | 93,0% | +0,4% / -2,6% |

### Ausführungsbeispiel 1b: Herstellung von Bendamustin

Das in Ausführungsbeispiel 1a erhaltene Rohprodukt Bendamustinethylester wurde in 210 ml 32% HCl gelöst und 2 h bei 40 °C Manteltemperatur gerührt. Die Lösung wurde auf Raumtemperatur abgekühlt, mit 8 g Aktivkohle versetzt, 30 min gerührt und über einen Sterilfilter filtriert. Die Salzsäure wurde destillativ entfernt und der Rückstand mit 400 ml Wasser/Aceton versetzt. Der Niederschlag wurde abfiltriert, mit Wasser und Ethylacetat gewaschen und getrocknet.

Dieser Versuch wurde zweimal durchgeführt. Es wurden folgende Ausbeuten an Bendamustin, jeweils bezogen auf das von Ethyl-4-(5-(bis-(2-hydroxyethyl)-amino)-1-methyl-1 H-benzo[d]imidazol-2-yl)butanoat ausgehende Verfahren, erhalten:

| V1b-1 | V1b-2 | Mittlere Ausbeute | Maximale Abweichung von mittlerer Ausbeute |
|---|---|---|---|
| 82,0% | 77,6% | 79,8% | +2,2% / -2,2% |

### Vergleichsbeispiel 2a: Herstellung von Bendamustinethylester

Ein alternatives Verfahren zur Herstellung von Bendamustinalkylester geht ebenfalls von einem Ethyl-4-(5-(bis-(2-hydroxyethyl)amino)-1-methyl-1 H-benzo[d]imidazol-2-yl)alkylester als Edukt aus. Dabei wird die Abgangstendenz der Hydroxylgruppen in diesem Edukt durch Einführung einer Aktivierungsgruppe (zum Beispiel einer p-Tosylgruppe, einer Mesylgruppe oder einer Triflatgruppe) erhöht. Der Austausch dieser Aktivierungsgruppe durch ein Halogenid gelingt anschließend unter milden Bedingungen, zum Beispiel durch Behandlung mit Lithiumchlorid in Dimethylformamid oder Ethanol.

In einem entsprechenden Versuch wurden 42,2 g Ethyl-4-(5-(bis-(2-hydroxyethyl)amino)-1-methyl-1H-benzo[d]imidazol-2-yl)butanoate) in 500 ml trockenem Dichlormethan gelöst und mit 41,6 ml Triethylamin (TEA) versetzt. Diese Lösung wurde langsam mit 52,2 g Methansulfonsäureanhydrid in 150 ml trockenem Dichlormethan versetzt und für 3 h bei 25 °C gerührt. Die Reaktionslösung wurde bei 30 °C im Vakuum von Lösungsmitteln befreit. Der erhaltene Rückstand wurde in 400 ml trockenem Ethanol gelöst, mit 30,5 g Lithiumchlorid versetzt und für 18 h bei 70 °C gerührt. Anschließend wurde Ethanol bei 40 °C destillativ entfernt und der Rückstand in 400 ml Dichlormethan (DCM) aufgenommen und mit 16,6 ml Triethylamin (TEA) versetzt. Nach der Zugabe von 200 ml Wasser wurde der pH-Wert mit konzentrierter K₂CO₃-Lösung auf 7,5 bis 9 justiert. Die entstandenen Phasen werden getrennt, die wässrige Phase mit weiteren 250 ml DCM extrahiert, die vereinigten organischen Phasen mit 100 ml halbkonzentrierter Natriumchlorid-Lösung gewaschen, über MgSO₄ getrocknet und im Vakuum vom Lösungsmittel befreit.

Dieser Versuch wurde viermal durchgeführt. Es wurden folgende Ausbeuten an Bendamustinethylester erhalten:

| V2a-1 | V2a-2 | V2a-3 | V2a-4 | Mittlere Ausbeute | Maximale Abweichung von mittlerer Ausbeute |
|---|---|---|---|---|---|
| 82,9% | 85,2% | 81,9% | 80,1% | 82,5% | +2,7%/-2,4% |

### Vergleichsbeispiel 2b: Herstellung von Bendamustin

Das in Vergleichsbeispiel 2a erhaltene Rohprodukt Bendamustinethylester wurde in 210 ml 32% HCl gelöst und für 2 h bei 40 °C Manteltemperatur gerührt. Die Lösung wurde auf Raumtemperatur abgekühlt, mit 8 g Aktivkohle versetzt, 30 min gerührt und über einen Sterilfilter filtriert. Die Salzsäure wurde destillativ entfernt und der Rückstand mit 400 ml Wasser/Aceton versetzt. Der Niederschlag wurde abfiltriert, mit Wasser und Ethylacetat gewaschen und getrocknet.

Dieser Versuch wurde zweimal durchgeführt. Es wurden folgende Ausbeuten an Bendamustin, jeweils bezogen auf das von Ethyl-4-(5-(bis-(2-hydroxyethyl)-amino)-1-methyl-1H-benzo[d]imidazol-2-yl)butanoat ausgehende Verfahren, erhalten:

| V2b-1 | V2b-2 | Mittlere Ausbeute | Maximale Abweichung von mittlerer Ausbeute |
|---|---|---|---|
| 69,8% | 71,3% | 70,6% | +0,5% / -0,8% |

### Vergleichsbeispiel 3a: Herstellung von Bendamustinethylester

Bendamustinethylester wurde hergestellt, indem dem Ausführungsbeispiel der DD 159877 gefolgt wurde. Demnach wurden 18,3 mol Thionylchlorid (2,175 kg) eingesetzt, um 22,3 mol Hydroxylgruppen (in 4,305 kg 4-[5-[Bis(2-Hydroxyethyl)amino]-1-methylbenzimidazol-2-yl]butansäureethylester) zu substituieren. Dabei kam es jedoch nur zu einer unvollständigen Umsetzung, wobei in der Reaktionsmischung nur etwa 47 Area-% des gewünschten Produktes enthalten waren.

Aus diesem Grund wurde die Thionylchloridmenge in weiteren Versuchen erhöht. Eine Verdreifachung der Thionylchloridmenge im Verhältnis zu 4-[5-[Bis(2-Hydroxyethyl)amino]-1-methylbenzimidazol-2-yl]butansäureethylester (von 0,82 Äquivalenten gemäß der DD 159877 auf 2,46 Äquivalente) führte zu 87 Area-% des gewünschten Produktes und stellte das Optimum der untersuchten Variationen dar.

Es wurden folgende Ausbeuten an Bendamustinethylester erhalten:

| V3a-1 | V3a-2 | Mittlere Ausbeute | Maximale Abweichung von mittlerer Ausbeute |
|---|---|---|---|
| 75,0% | 59,0% | 67,0% | +8,0% -8,0% |

Das gemäß der vorstehenden Vorgehensweise erhaltene Material genügte trotz zweifacher Umkristallisation nicht den Spezifikationsanforderungen der ICH-Guidelines.

Diese Versuche zeigen, dass das erfindungsgemäße Verfahren gegenüber den aus dem Stand der Technik bekannten Verfahren überraschenderweise eine deutlich erhöhte, reproduzierbare Ausbeute liefert.

## Patentansprüche

1. Verfahren zur Herstellung einer Verbindung, die durch die Formel (I) 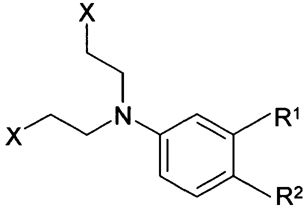 dargestellt ist, oder einem Salz davon, wobei X für einen Halogenrest steht, R¹ und R² so gewählt sind, dass (1) R¹ H ist und R² CH₂(CH₂)ₘCOOR³ ist, (2) R¹ CH₂(CH₂)ₘCOOR³ ist und R² H ist oder (3) R¹ und R² zusammen für einen Rest stehen, der durch die Formel (II) 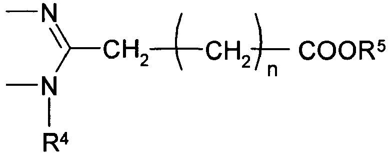 dargestellt ist, wobei R³, R⁴ und R⁵ unabhängig voneinander für einen Alkylrest stehen, und wobei m und n unabhängig voneinander eine Zahl im Bereich von 0-12 darstellen, **dadurch gekennzeichnet, dass** man
eine Verbindung, die durch die Formel (III) 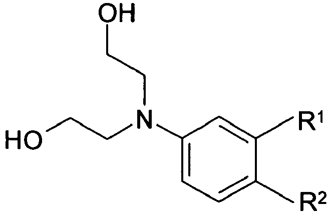 dargestellt ist, mit einer Mischung zur Reaktion bringt, die eine Verbindung (i), die aus der Gruppe ausgewählt ist, die aus einer Verbindung, die durch die Formel (IV) 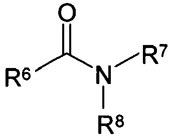 dargestellt ist, wobei R⁶ für H oder einen Alkylrest steht, und R⁷ und R⁸ unabhängig voneinander für einen Alkylrest stehen, und einer Verbindung, die durch die Formel (V) 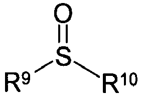 dargestellt ist, wobei R⁹ und R¹⁰ unabhängig voneinander für einen Alkylrest stehen, besteht, und eine Verbindung (ii), die durch die Formel (VI) 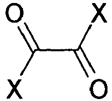 dargestellt ist, enthält.

2. Verfahren nach Anspruch 1, **dadurch gekennzeichnet, dass** X ein Chlorid darstellt.

3. Verfahren nach Anspruch 1 oder 2, **dadurch gekennzeichnet, dass** R¹ und R² zusammen für einen Rest stehen, der durch die Formel (II) dargestellt ist.

4. Verfahren nach Anspruch 3, **dadurch gekennzeichnet, dass** n eine ganze Zahl im Bereich von 1-3 ist.

5. Verfahren nach Anspruch 4, **dadurch gekennzeichnet, dass** R⁴ für einen Alkylrest steht, der aus der Gruppe ausgewählt ist, die aus einem Methylrest, einem Ethylrest und einem Propylrest besteht.

6. Verfahren nach Anspruch 5, **dadurch gekennzeichnet, dass** R⁴ für einen Methylrest steht.

7. Verfahren nach einem der Ansprüche 1-6, **dadurch gekennzeichnet, dass** R⁵ für einen Alkylrest steht, der aus der Gruppe ausgewählt ist, der aus einem Methylrest, einem Ethylrest, einem Propylrest und einem Butylrest besteht.

8. Verfahren nach Anspruch 1 oder 2, **dadurch gekennzeichnet, dass** R¹ H ist und R² CH₂(CH₂)ₘCOOH ist.

9. Verfahren nach Anspruch 8, **dadurch gekennzeichnet, dass** m eine ganze Zahl im Bereich von 1 - 3 ist.

10. Verfahren nach Anspruch 9, **dadurch gekennzeichnet, dass** m gleich 2 ist.

11. Verfahren nach einem der Ansprüche 1-11, **dadurch gekennzeichnet, dass** es sich bei der Verbindung, die durch die Formel (IV) dargestellt ist, um ein Dialkylformamid handelt.

12. Verfahren nach Anspruch 11, **dadurch gekennzeichnet, dass** es sich bei dem Dialkylformamid um Dimethylformamid handelt.

13. Verfahren nach einem der Ansprüche 1-12, **dadurch gekennzeichnet, dass** es sich bei der Verbindung, die durch die Formel (IV) dargestellt ist, um Dimethylacetamid handelt.

14. Verfahren nach einem der Ansprüche 1-12, **dadurch gekennzeichnet, dass** es sich bei der Verbindung, die durch die Formel (V) dargestellt ist, um Dimethylsulfoxid handelt.

15. Verfahren nach einem der Ansprüche 1-14, **dadurch gekennzeichnet, dass** es sich bei der Verbindung, die durch die Formel (VI) dargestellt ist, um Oxalylchlorid handelt.

16. Verfahren nach einem der Ansprüche 1-15, **dadurch gekennzeichnet, dass** das molare Verhältnis von der Verbindung gemäß Formel (VI) zur Verbindung gemäß Formel (III) wenigstens 1,3 beträgt.

17. Verfahren nach einem der Ansprüche 1 - 16, **dadurch gekennzeichnet, dass** die Reaktion in einem chlorierten Lösungsmittel oder einem aprotischen Lösungsmittel durchgeführt wird.

18. Verfahren zur Herstellung einer Verbindung, die durch die Formel (VII) 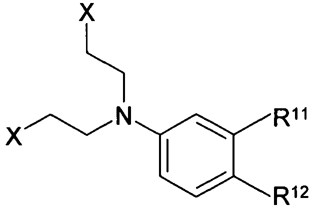 dargestellt ist, oder einem Salz davon, wobei X für einen Halogenrest steht, R¹¹ und R¹² so gewählt sind, dass (1) R¹¹ H ist und R¹² CH₂(CH₂)ₘCOOH ist, (2) R¹¹ CH₂(CH₂)ₘCOOH ist und R¹² H ist oder (3) R¹¹ und R¹² zusammen für einen Rest stehen, der durch die Formel (VIII) 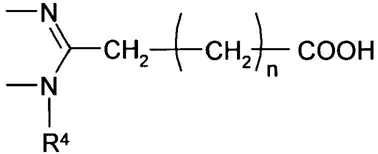 dargestellt ist, wobei R⁴ für einen Alkylrest steht und wobei m und n unabhängig voneinander eine Zahl im Bereich von 0-12 darstellen,
**dadurch gekennzeichnet, dass** man das Verfahren gemäß Anspruch 1 durchführt und die erhaltene Esterverbindung, die durch die Formel (I) dargestellt ist, einer Esterhydrolyse unterzieht.

## Claims

1. Method for the production of a compound represented by formula (I) 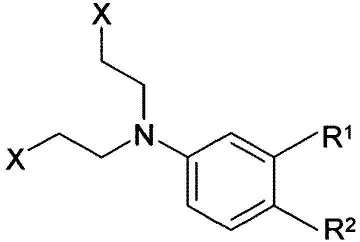 or a salt thereof, whereby X represents a halogen residue, R¹ and R² are selected appropriately such that (1) R¹ is H and R² is CH₂(CH₂)ₘCOOR³, (2) R¹ is CH₂(CH₂)ₘCOOR³ and R² is H or (3) R¹ and R² together represent a residue that is represented by formula (II) 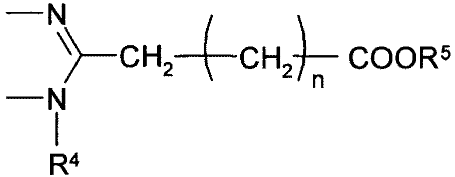 whereby R³, R⁴ and R⁵, independent of each other, represent an alkyl residue, and whereby m and n, independent of each other, represent a number in the range of 0 - 12,
**characterised in that**
a compound represented by formula (III) 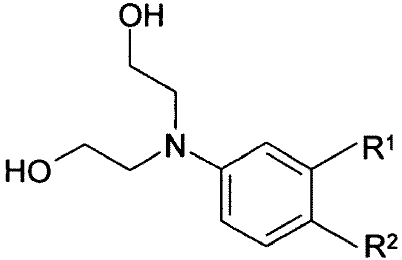 Is reacted with a mixture that contains a compound (i) selected from the group consisting of a compound represented by formula (IV) 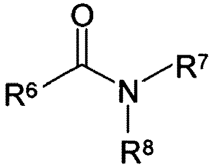 whereby R⁶ represents H or an alkyl residue, and R⁷ and R⁸, independent of each other, represent an alkyl residue, and a compound represented by formula (V) 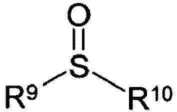 whereby R⁹ and R¹⁰, independent of each other, represent an alkyl residue, and a compound (ii) represented by formula (VI).

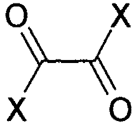

2. Method according to claim 1, **characterised in that** X is a chloride.

3. Method according to claim 1 or 2, **characterised in that** R¹ and R² together represent a residue that is represented by formula (II).

4. Method according to claim 3, **characterised in that** n is an integer in the range of 1 - 3.

5. Method according to claim 4, **characterised in that** R⁴ represents an alkyl residue selected from the group consisting of a methyl residue, an ethyl residue, and a propyl residue.

6. Method according to claim 5, **characterised in that** R⁴ is a methyl residue.

7. Method according to any one of the claims 1 - 6, **characterised in that** R⁵ represents an alkyl residue selected from the group consisting of a methyl residue, an ethyl residue, a propyl residue, and a butyl residue.

8. Method according to claim 1 or 2, **characterised in that** R¹ is H and R² is CH₂(CH₂)ₘCOOH.

9. Method according to claim 8, **characterised in that** m is an integer in the range of 1 - 3.

10. Method according to claim 9, **characterised in that** m is equal to 2.

11. Method according to any one of the claims 1-11, **characterised in that** the compound represented by formula (IV) is a dialkylformamide.

12. Method according to claim 11, **characterised in that** the dialkylformamide is dimethylformamide.

13. Method according to any one of the claims 1-12, **characterised in that** the compound represented by formula (IV) is dimethylacetamide.

14. Method according to any one of the claims 1-12, **characterised in that** the compound represented by formula (V) is dimethylsulfoxide.

15. Method according to any one of the claims 1-14, **characterised in that** the compound represented by formula (VI) is oxalylchloride.

16. Method according to any one of the claims 1-15, **characterised in that** the molar ratio of the compound according to formula (VI) and the compound according to formula (III) is at least 1.3.

17. Method according to any one of the claims 1-16, **characterised in that** the reaction is made to take place in a chlorinated solvent or in an aprotic solvent.

18. Method for the production of a compound represented by formula (VII) 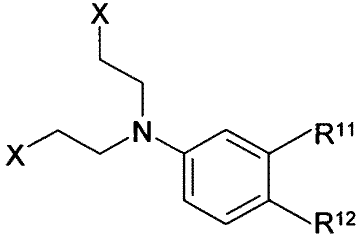 or a salt thereof, whereby X represents a halogen residue, R¹¹ and R¹² are selected appropriately such that (1) R¹¹ is H and R¹² is CH₂(CH₂)ₘCOOH, (2) R¹¹ is CH₂(CH₂)ₘCOOH and R¹² is H or (3) R¹¹ and R¹² together represent a residue that is represented by formula (VIII) 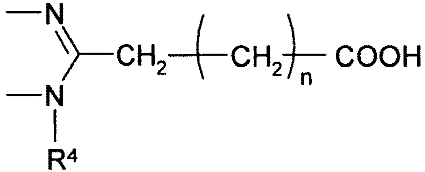 whereby R⁴ represents an alkyl residue, and whereby m and n, independent of each other, represent a number in the range of 0 - 12,
**characterised in that** the process according to claim 1 is made to take place and the ester compound thus obtained, which is represented by formula (I), is subjected to an ester hydrolysis.

## Revendications

1. Procédé de fabrication d'un composé qui est représenté par la formule (I) 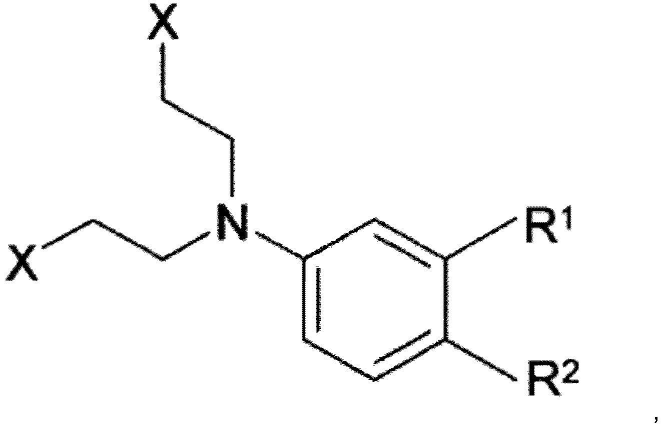 ou d'un sel de celui-ci, dans laquelle X représente un résidu halogène, R¹ et R² sont choisis de sorte que (1) R¹ est H et R² est CH₂(CH₂)ₘCOOR³, (2) R¹ est CH₂(CH₂)ₘCOOR³ et R² est H ou (3) R¹ et R² représentent ensemble un résidu qui est représenté par la formule (II) 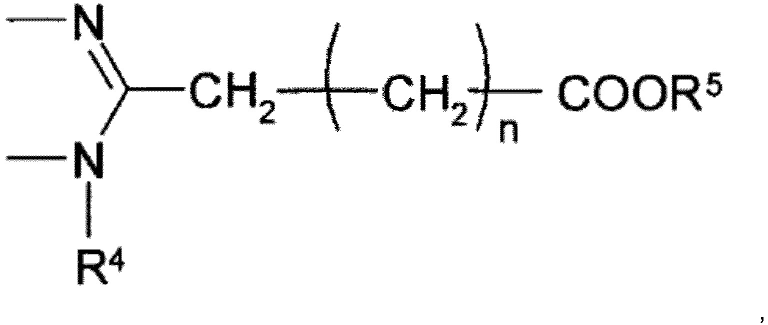 dans laquelle R³, R⁴ et R⁵ représentent indépendamment les uns des autres un résidu alkyle, et dans laquelle m et n représentent indépendamment l'un de l'autre un nombre dans la plage de 0 à 12, **caractérisé en ce qu'**on met à réagir un composé qui est représenté par la formule (III) 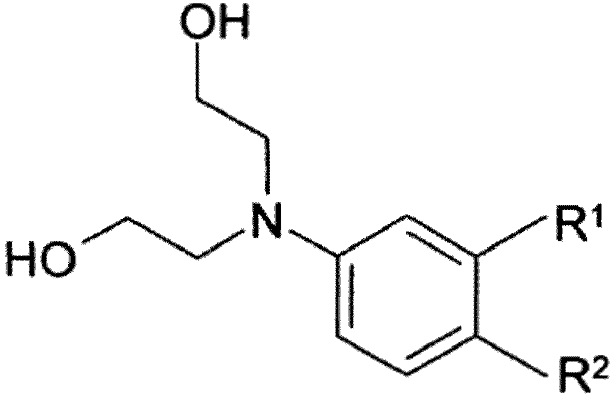 avec un mélange qui contient un composé (i) qui est choisi parmi le groupe qui se compose d'un composé qui est représenté par la formule (IV) 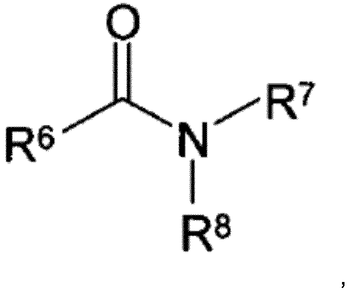 dans laquelle R⁶ représente H ou un résidu alkyle, et R⁷ et R⁸ représentent indépendamment l'un de l'autre un résidu alkyle, et d'un composé qui est représenté par la formule (V) 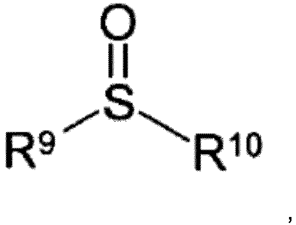 dans laquelle R⁹ et R¹⁰ représentent indépendamment l'un de l'autre un résidu alkyle, et un composé (ii) qui est représenté par la formule (VI)

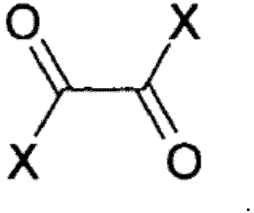

2. Procédé selon la revendication 1, **caractérisé en ce que** X représente un chlorure.

3. Procédé selon la revendication 1 ou 2, **caractérisé en ce que** R¹ et R² représentent ensemble un résidu qui est représenté par la formule (II).

4. Procédé selon la revendication 3, **caractérisé en ce que** n est un nombre entier dans la plage de 1 à 3.

5. Procédé selon la revendication 4, **caractérisé en ce que** R⁴ représente un résidu alkyle qui est choisi parmi le groupe qui se compose d'un résidu méthyle, d'un résidu éthyle et d'un résidu propyle.

6. Procédé selon la revendication 5, **caractérisé en ce que** R⁴ représente un résidu méthyle.

7. Procédé selon une des revendications 1 à 6, **caractérisé en ce que** R⁵ représente un résidu alkyle qui est choisi parmi le groupe qui se compose d'un résidu méthyle, d'un résidu éthyle, d'un résidu propyle et d'un résidu butyle.

8. Procédé selon la revendication 1 ou 2, **caractérisé en ce que** R¹ est H et R² est CH₂(CH₂)ₘCOOH.

9. Procédé selon la revendication 8, **caractérisé en ce que** m est un nombre entier dans la plage de 1 à 3.

10. Procédé selon la revendication 9, **caractérisé en ce que** m est égal à 2.

11. Procédé selon une des revendications 1 à 11, **caractérisé en ce qu'**il s'agit pour le composé qui est représenté par la formule (IV) d'un dialkylformamide.

12. Procédé selon la revendication 11, **caractérisé en ce qu'**il s'agit pour le dialkylformamide du diméthylformamide.

13. Procédé selon une des revendications 1 à 12, **caractérisé en ce qu'**il s'agit pour le composé qui est représenté par la formule (IV) du diméthylacétamide.

14. Procédé selon une des revendications 1 à 12, **caractérisé en ce qu'**il s'agit pour le composé qui est représenté par la formule (V) du diméthylsulfoxyde.

15. Procédé selon une des revendications 1 à 14, **caractérisé en ce qu'**il s'agit pour le composé qui est représenté par la formule (VI) du chlorure d'oxalyle.

16. Procédé selon une des revendications 1 à 15, **caractérisé en ce que** le rapport molaire du composé selon la formule (VI) sur le composé selon la formule (III) se monte à au moins 1,3.

17. Procédé selon une des revendications 1 à 16, **caractérisé en ce que** la réaction est réalisée dans un solvant chloré ou un solvant aprotique.

18. Procédé de fabrication d'un composé qui est représenté par la formule (VII) 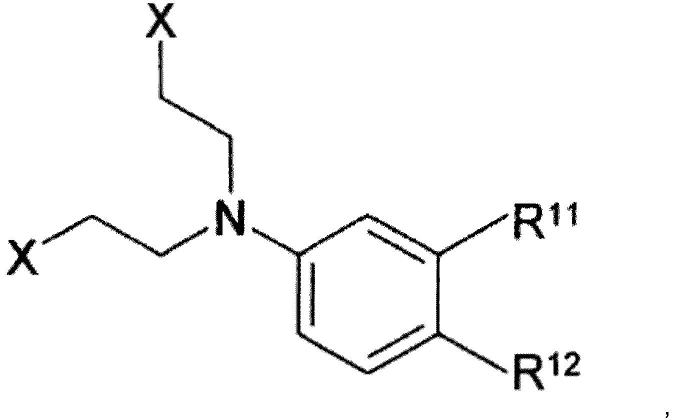 ou d'un sel de celui-ci, dans laquelle X représente un résidu halogène, R¹¹ et R¹² sont choisis de sorte que (1) R¹¹ est H et R¹² est CH₂(CH₂)ₘCOOH, (2) R¹¹ est CH₂(CH₂)ₘCOOH et R¹² est H ou (3) R¹¹ et R¹² représentent ensemble un résidu qui est représenté par la formule (VIII) 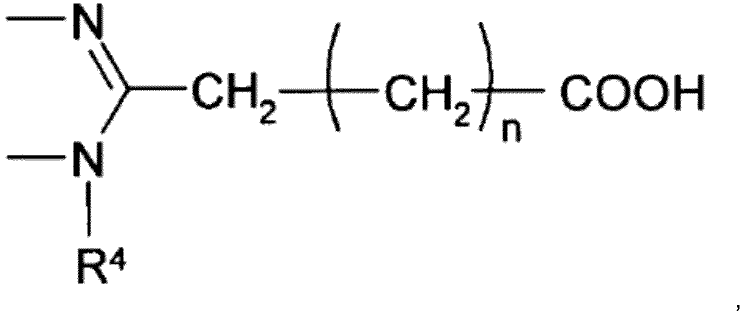 dans laquelle R⁴ représente un résidu alkyle, et dans laquelle m et n représentent indépendamment l'un de l'autre un nombre dans la plage de 0 à 12,
**caractérisé en ce qu'**on réalise le procédé selon la revendication 1 et le composé d'ester obtenu qui est représenté par la formule (I) subit une hydrolyse d'ester.
